# EUROPEAN PATENT APPLICATION

(11) **EP 4 163 371 A1**
(43) Date of publication of application: **12.04.2023**
(21) Application number: 21818014.9
(22) Date of filing: 31.05.2021
(51) Int. Cl.: C12N 15/10

(54) **NUCLEIC ACID EXTRACTION METHOD AND APPLICATION**

(30) Priority: 01.06.2020 CN 202010488101
(71) Applicant: Nuhigh Biotechnologies Co., Ltd, Suzhou, Jiangsu 215123 (CN)
(72) Inventor: FU, Dongke, Suzhou City, Jiangsu 215123 (CN); HE, Wenlong, Suzhou City, Jiangsu 215123 (CN); LIU, Liandi, Suzhou City, Jiangsu 215123 (CN); BI, Wanli, Suzhou City, Jiangsu 215123 (CN)
(74) Representative: Gill Jennings & Every LLP
(86) International application number: PCT/CN2021/097213
(87) International publication number: WO 2021/244469

(57) **Abstract**

Disclosed is a nucleic acid extraction method. By using a quaternary ammonium salt, nucleic acid molecules in a solution are adsorbed to adsorbing materials such as silicon beads, magnetic beads, diatomite or silica gel or precipitated, thereby improving efficiency of nucleic acid purification and recovery.

## Description

### TECHNICAL FIELD

The invention relates to the technical field of biotechnology, and more specifically to a nucleic acid extraction method and application.

### BACKGROUND OF THE INVENTION

Nucleic acid is the carriers of genetic information and is usually present in various biological samples, such as blood, serum, tissue, cell, saliva, feces. Since the biological sample contains a large number of biological molecules (such as proteins, lipids, and sugars) and inorganic ions, in addition to nucleic acids, it cannot be directly used for further molecular biology experiments and detection. Accordingly, separation and purification are required to remove other impurities out of the nucleic acid, and this process is also referred to as nucleic acid extraction.

The extracted nucleic acids, especially high molecular weight DNA (deoxyribonucleic acid), have many applications in molecular biology, biotechnology and clinical research. The methods, for example, PCR (polymerase chain reaction), DNA hybridization, restriction enzyme digestion, DNA sequencing, and microarray experiments, can be used to study SNP (Single Nucleotide Polymorphism) for clinical applications such as genetic disease screening, tumor detection, and individualized medication guidance, and study STR (Short Tandem Repeat) for forensic applications such as individual identification and paternity identification. In addition, the methods can also be used for genetic researches such as population screening and phylogeny.

The methods of separating and extracting nucleic acid have been reported in the literatures (e.g. Chapter 2 (DNA) and Chapter 4 (RNA) of F. Ausubel et al., eds., Current Protocols in Molecular Biology, Wiley-Interscience, New York, 1993). These methods generally require resuspending the sample in a certain solution and rupturing the cells through a chemical reagent or an enzymatic method to release nucleic acid. This process is referred to as lysis. The released nucleic acid can reversibly bind to the nucleic acid-adsorbing material in solution or precipitate in the presence of polar organic solvent. Such materials comprise glass particle, glass fiber, magnetic bead, diatomite, silica gel, etc., or variations or combinations of the above materials. High concentrations of chaotropic salts can facilitate the binding of nucleic acids to the above materials. Polar organic solvents typically comprise absolute ethanol, isopropanol, polyethylene glycol (PEG), and the like. Common chaotropic salts comprise sodium chloride, sodium acetate, guanidine hydrochloride, guanidine isothiocyanate, and the like. The binding between nucleic acid and the above adsorbing materials involves hydrogen bond, hydrophobic interaction, electrostatic interaction, etc. Such a process is referred to as binding of nucleic acid. Then, impurities such as salt ions and proteins are washed off by using specific washing solutions, such as 75-80% ethanol. Such a process is referred to as rinsing. Finally, under the action of eluents, the nucleic acid molecules are separated from the above materials and collected into the eluents. Such a process is referred to as elution. The eluent herein can be low-salt buffer or deionized ultrapure water.

In the process of nucleic acid purification and separation, most of nucleic acid extraction reagents and methods on the market currently use guanidine salts as chaotropic salts. Guanidine salts have the effect of denaturing proteins, and thus can lyse biological samples and release nucleic acid molecules. Meanwhile, guanidine salts can neutralize the negative charge of nucleic acid molecules such that the nucleic acid molecules can reversibly bind to the materials such as silica gel and magnetic beads, and thus can make nucleic acid molecules be adsorbed on these materials. Then, impurities such as protein and salt ions can be removed by washing, and the target nucleic acid molecules can be eluted under certain conditions, so as to achieve the purpose of separating and purifying nucleic acid.

However, guanidine salt has poor stability and higher light sensitivity, and easily change color and decompose, for example, guanidine hydrochloride easily decomposes into ammonia and urea in water. The types of guanidine salts which can be selected are relatively few, and the commonly available guanidine salt is guanidine hydrochloride or guanidine isothiocyanate. During DNA extraction, it is usually necessary to add ethanol or isopropanol to facilitate DNA precipitation; nevertheless, guanidine salts have low solubility in ethanol and isopropanol and thus usually result in overly large volume of binding solution, which is unfavorable for the extraction of trace DNA.

### SUMMARY OF THE INVENTION

In view of the above, the purpose of the present invention is to provide application of quaternary ammonium salts in nucleic acid extraction, such that the quaternary ammonium salts are used as chaotropic salts for nucleic acid extraction, and nucleic acid molecules in the solution are adsorbed to adsorbing materials such as silicon beads, magnetic beads, diatomite or silica gel, or are precipitated, which can effectively improve the efficiency of nucleic acid purification and recovery, and can be used for plasmid DNA extraction, viral genome extraction, bacterial genome extraction, as well as animal, plant and human genome DNA extraction.
Another purpose of the present invention is to provide a nucleic acid extraction reagent with a quaternary ammonium salt as a chaotropic salt;
Another purpose of the present invention is to provide a nucleic acid extraction method using a quaternary ammonium salt as a chaotropic salt.

For realizing the above inventive purpose, the present invention provides the following technical solutions.

Use of quaternary ammonium salt in nucleic acid extraction, wherein the quaternary ammonium salt has the structure as shown in formula I, 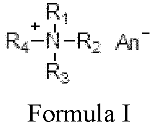 wherein An⁻ is an anion, and R₁, R₂, R₃, and R₄ are independently selected from alkyl groups.

The quaternary ammonium salts are used as chaotropic salts for nucleic acid extraction in the present invention. As compared with guanidine salts as chaotropic salts, the quaternary ammonium salts are more stable, include more kinds to be selected, and have higher solubility in organic solvents such as ethanol, showing that the quaternary ammonium salts have a lower cost at the same working concentration.

Preferably, the anion is a halogen anion, and further preferably, the anion is a chloride ion or a bromide ion.

Preferably, R₁, R₂, R₃, and R₄ are independently selected from C₁-C₅ alkyls, and further preferably, R₁, R₂, R₃, and R₄ are independently selected from methyl, ethyl, propyl, butyl, and pentyl.

In the specific embodiment of the present invention, the quaternary ammonium salt is tetramethylammonium bromide, tetramethylammonium chloride, tetraethylammonium bromide, tetraethylammonium chloride, tetrapropylammonium bromide, tetrapropylammonium chloride, tetrabutylammonium bromide, tetrabutylammonium chloride, tetrapentylammonium bromide, or tetrapentylammonium chloride.

The quaternary ammonium salts are used as chaotropic salts in nucleic acid extraction. A certain concentration of quaternary ammonium salts can neutralize the negative charge of nucleic acids, such that the nucleic acids can bind to the adsorbing material or be precipitated in the presence of polar solvents (ethanol, isopropanol, etc.) by hydrogen bond, hydrophobic interaction and electrostatic interaction. The quaternary ammonium salt functions in the nucleic acid extraction in the form of a solution, and the salt concentration range of the quaternary ammonium salt solution is 100 mM - 2.5 M, preferably 250 mM - 2.5 M. In a specific embodiment of the present invention, the concentration of the quaternary ammonium salt is 250 mM, 1.6 M or 2.5 M.

The nucleic acid in the present invention refers to a biological macromolecule formed by the polymerization of many nucleotides, which is an extremely important living substance. Nucleic acids widely exist in animal and plant cells and microorganisms. According to the chemical composition, nucleic acid is mainly divided into deoxyribonucleic acid (DNA) and ribonucleic acid (RNA), and the nucleic acid molecule in the present invention also refers to DNA, RNA or a mixture thereof.

Preferably, according to the extraction means, the nucleic acid extraction can be divided into phenol-chloroform extraction method, polar solvent (ethanol, isopropanol or polyethylene glycol) precipitation method, and solid-phase support adsorption method. The solid-phase support adsorption method is to utilize the strong affinity and adsorption force of a support to nucleic acids to specifically bind the released nucleic acid to the specific support. Some commonly used supports comprise silica beads, magnetic beads, diatomite or silica gel, etc. According to the type of nucleic acid, the nucleic acid extraction can be divided into plasmid DNA extraction, viral genome extraction (such as lambda DNA extraction), bacterial genome extraction (such as *E. coli* DNA extraction), and animal, plant and human genome DNA extraction. The extraction of these types of nucleic acid can select a suitable extraction means in view of the prior art.

According to the above uses provided by the present invention, the present invention also provides a nucleic acid extraction reagent, wherein the chaotropic salt is the quaternary ammonium salt of the structure as shown in formula I of the present invention.

Preferably, the nucleic acid extraction reagent comprises the quaternary ammonium salt of the structure as shown in formula I of the present invention, and one or more components of a lysis solution, a binding solution, a washing solution, an eluent, and an adsorbing material; and the quaternary ammonium salt can be used independently as a component of the nucleic acid extraction reagent, or added to the lysis solution, or added to the binding solution.

The lysis solution, binding solution, washing solution, adsorbing material, and eluent can be combined in different combinations according to different extraction means or different types of nucleic acids to be extracted. For example, in the extraction of human genome, plasmid DNA and trace genomic DNA, the nucleic acid extraction reagents comprises a lysis solution, a binding solution, a washing solution, an adsorbing material, and an eluent; in the extraction of viral genome-lambda DNA, the nucleic acid extraction reagent comprises a lysis solution, a washing solution, an adsorbing material, and an eluent. Sometimes, the binding solution can be added directly to a lysis solution, which is referred to as a lysis binding solution, such as in the extraction of viral genome-lambda DNA.

According to different nucleic acid extractions, the lysis solution comprises one or more of buffer (10-50 mM), protease (0.5 mg/ml), alkali (200 mM), surfactant (1-10%), nucleic acid stabilizer (36-50 mM), lysozyme (5 mg/ml), and phenol-chloroform-isopentanol, such as buffer + protease + surfactant + nucleic acid stabilizer, buffer + surfactant, buffer + nucleic acid stabilizer + surfactant + alkali, buffer + lysozyme + protease + phenol-chloroform-isopentanol, buffer + nucleic acid stabilizer + surfactant + protease, etc. In a specific embodiment of the present invention, the protease is proteinase K, the surfactant is SDS, PEG or SLS, the alkali is sodium hydroxide, the buffer is phosphate buffer or Tris-HCl, and the nucleic acid stabilizer is EDTA;

Preferably, the binding solution is isopropanol, absolute ethanol or polyethylene glycol.

Preferably, the washing solution is 75-80 % ethanol solution; the eluent can be a low-salt buffer (10 mM, such as Tris-HCl) or deionized ultrapure water, and further preferably, the eluent may also comprises surfactant or nucleic acid stabilizer (1 mM), wherein the surfactant is preferably SDS or TritonX-100 and the mass percentage concentration is 0.1-1%, and the nucleic acid stabilizer is preferably EDTA and the concentration is 1 mM.

In addition, the present invention also provides a nucleic acid extraction method, wherein the quaternary ammonium salt of the structure as shown in formula I of the present invention is added as a chaotropic salt for extraction during the nucleic acid extraction process, and other reagents can be conventionally selected.

Further, the extraction method comprises adding a lysis solution to the sample to be extracted for lysing the sample and releasing nucleic acid molecules, and then adding a binding solution to bind the nucleic acid to the adsorbing material or precipitate the nucleic acid, wherein the quaternary ammonium salt is added after the lysis or after addition of the binding solution, or added to the lysis solution first and then added together, or added to the binding solution first and then added together.

Preferably, the extraction method further comprises using a washing solution to wash the nucleic acid on the adsorbing material or the precipitated nucleic acid.

Preferably, the extraction method further comprises using an eluent to elute the nucleic acid on the adsorbing material, or using an eluent to dissolve the precipitated nucleic acid.

From the above technical solutions, it can be seen that the present invention provides use of a quaternary ammonium salt with a structure as shown in formula I as a chaotropic salt in nucleic acid extraction, in place of the commonly used guanidine salt, wherein in the presence of a binding solution of polar organic solvent, the quaternary ammonium salt could make the nucleic acid molecules be adsorbed to the materials such as silica beads, magnetic beads, diatomite or silica gel, or be precipitated, and thus can effectively improve the efficiency of nucleic acid purification and recovery; the quaternary ammonium salt can be used for plasmid DNA extraction, viral genome extraction, bacterial genome extraction, or animal, plant and human genome DNA extraction.

### BRIEF DESCRIPTION OF THE DRAWINGS

**FIG. 1** shows the comparison of real-time PCR amplification curves of DNAs extracted from human blood genome.
**FIG. 2** shows the agarose gel electrophoretogram of the extraction of lambda DNA.
**FIG. 3** shows the agarose gel electrophoretogram of the extraction of plasmid DNA.
**FIG. 4** shows the agarose gel electrophoretogram of the extraction of *E. coli* genome.
**FIG. 5** shows the STR-capillary electrophoretogram of trace genomic DNA.
**FIG. 6** shows the comparison of the effects of CTAB and TEAB on trace genome extraction.
**FIG. 7** shows the real-time PCR amplification curve of the extraction of nucleic acid of MERS pseudovirus.

### DETAILED DESCRIPTION OF THE INVENTION

The present invention discloses a nucleic acid extraction method and use, and those skilled in the art could appropriately improve the process parameters in view of the disclosures of the present application. It should be particularly pointed out that all similar substitutions and modifications are obvious to those skilled in the art, and deemed to be included in the present invention. The extraction method and use of the present invention have been described through the preferred examples, and relevant persons can obviously modify or appropriately change and combine the extraction method and use of the present application without departing from the content, spirit and scope of the present invention, so as to realize and apply the technology of the present invention.

In the specific embodiment of the present invention, the raw materials used in each treatment group of the comparative experiments provided are the same, and the other experiment conditions of each group are also the same except for the due differences. The raw materials, reagents, etc. involved in the present invention can be commercially available unless otherwise specified.

The present invention is further illustrated in view of the Examples below.

### Example 1: Extraction and purification of DNA from human blood

One group of extraction solutions containing tetraethylammonium chloride was used to extract nucleic acid by magnetic bead method, while another group of extraction solutions containing the same components except tetraethylammonium chloride was used as a control, to compare the extraction effects of the two solutions. The reagent components used are as follows:
Extraction solution 1 (lysis solution) comprises Tris-HCl (10 mM), EDTA (50 mM), SDS (1%), tetraethylammonium chloride (250 mM), and proteinase K (0.5 mg/ml);
Extraction solution 2 (binding solution) comprises 100% isopropanol;
Extraction solution 3 (washing solution) comprises 75% ethanol;
Extraction solution 4 (eluent) comprises ultrapure water; and
Magnetic bead suspension comprises superparamagnetic nanomagnetic beads dispersed in ultrapure water.

The preparation method comprises the following steps:
1. 5 µl of human whole blood was taken and placed into a centrifuge tube, and 300 µl of Extraction solution 1 was added to treat the blood at 56°C for 15 minutes;
2. 300 µl of Extraction solution 2 and 400 µg of the magnetic bead suspension were added, and the mixture was shaken and mixed well at 800 rpm for 10 minutes;
3. The centrifuge tube was placed on a magnetic stand for magnetic separation, the liquid in the tube was sucked and discarded, 500 µl of Extraction solution 3 was added, the mixture was shaken and mixed well for 1 minute, and the supernatant was discarded after magnetic separation; step 3 was repeated once;
4. After the above magnetic beads were dried at room temperature until there was no obvious ethanol odor, 50 µl of Extraction solution 4 was added to treat the beads at 65°C for 10 minutes; and
5. The supernatant was collected after magnetic separation.

### The collected supernatant above was amplified by real-time PCR:

25 µl of amplification system comprises the following components: 12.5 µl 2 × PCR Mix, l µl of each primer (10 µM), 3 µl of the collected supernatant, and 7.5 µl pure water, wherein in addition to the two groups of bloods to be extracted, the templates also comprise the standard l ng human genome as a positive control and Extraction solution 4 as a negative control.
The thermal cycle is as follows: 95°C, 10 minutes; (95°C, 15 seconds; 60°C, 45 seconds) x 40 cycles

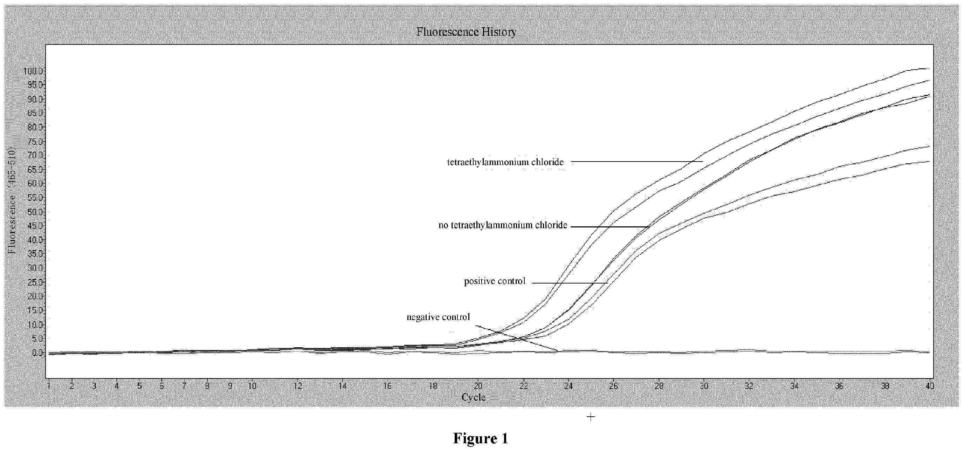

The results were shown in Figure 1. The results showed that when the blood DNA was extracted by using magnetic bead method and amplified, the effect of extraction and amplification by adding tetraethylammonium chloride was obviously better than that without tetraethylammonium chloride, indicating that tetraethylammonium chloride could improve the precipitation recovery efficiency of DNA. It also showed that the extraction solution containing quaternary ammonium salts could effectively extract and purify DNA in blood.

### Example 2: Extraction and purification of lambda DNA

This Example aims to illustrate the effect of quaternary ammonium salts on nucleic acid adsorption, and thus lambda DNA was used for simulating purification. One group of extraction solutions containing tetrapropylammonium bromide was used for nucleic acid extraction, while another group of an extraction solution containing the same components without tetrapropylammonium bromide was used as a control to compare the extraction effects of the two groups. The components of the reagents used are as follows:
Extraction solution 1 (lysis binding solution) comprises Tris-HCl (20 mM), tetrapropylammonium bromide (250 mM), and 10% PEG 8000;
Extraction solution 2 (washing solution) comprises 75% ethanol;
Extraction solution 3 (eluent) comprises ultrapure water; and
Magnetic bead suspension comprises superparamagnetic nanomagnetic beads dispersed in ultrapure water.

The preparation method comprises the following steps:
1. 50 ng lambda DNA was taken and placed in a centrifuge tube, 400 µl of Extraction solution 1 was added, and the mixture was shaken and mixed well at 500 rpm-12000 rpm for 10 minutes;
2. The centrifuge tube was placed on a magnetic stand for magnetic separation, and the liquid in the tube was sucked and discarded, 500 µl of Extraction solution 2 was added, the mixture was shaken and mixed well for 1 minute, and the supernatant was discarded after magnetic separation; step 3 was repeated once;
3. After the magnetic beads were dried at room temperature until there was no obvious ethanol odor, 50 µl of Extraction solution 4 was added to treat the beads at 65°C for 10 minutes; and
4. The supernatant was collected after magnetic separation.

### The collected supernatant above was amplified by PCR:

20 µl of amplification system comprises the following components: 10 µl 2 × PCR Mix, l µl of each primer (10 µM), and 8 µl of the collected supernatant, wherein in addition to the two groups of blood to be extracted, the templates also comprise the standard 10 ng lambda DNA as a positive control and Extraction solution 3 as a negative control.
The thermal cycle is as follows: 95°C, 5 minutes; (95°C, 20 seconds; 60°C, 3 minutes) x 35 cycles

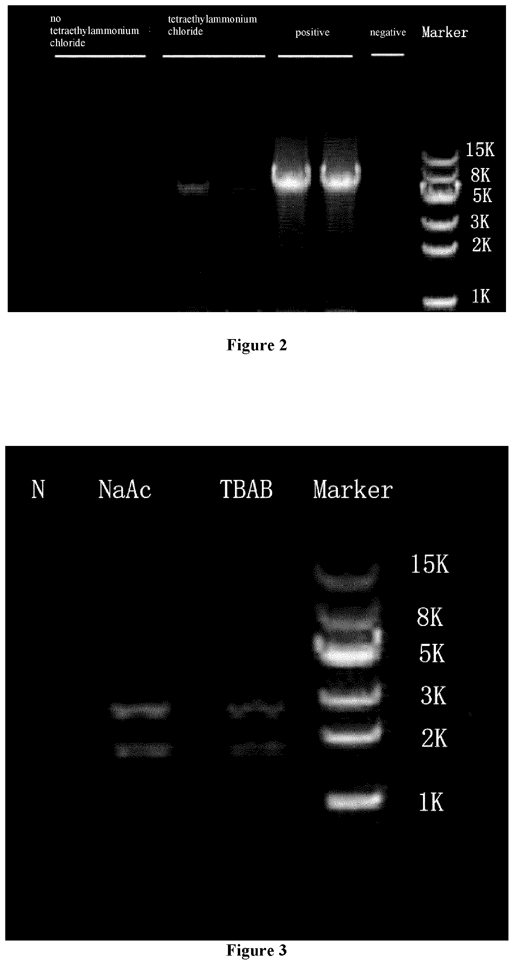

The electrophoresis results were shown in Figure 2.

The results showed that when the lambda DNA was extracted by using the above extraction solution and amplified, there is a band at 6000 bp for the group with tetrapropylammonium bromide, which is obviously better than that without tetrapropylammonium bromide, indicating that tetrapropylammonium bromide could improve the precipitation recovery efficiency of DNA.

### Example 3: Extraction and purification of plasmid DNA

The present invention uses quaternary ammonium salts in the method for ethanol precipitation of nucleic acid, which could effectively extract *E*. *coli* plasmids and comprise the following five components: Extraction solution 1, Extraction solution 2, Extraction solution 3, Extraction solution 4, and Extraction solution 5.
Extraction solution 1 (lysis solution) comprises 10 mM Tris-HCl (pH 8.0) and 50 mM EDTA(pH 8.0);
Extraction solution 2 (lysis solution) comprises 200 mM NaOH and 1% SDS;
Extraction solution 3 (chaotropic salt) comprises 2.5 M tetrabutylammonium bromide (pH 7.0);
Extraction solution 4 (binding solution) comprises 100% isoproterenol;
Extraction solution 5 (washing solution) comprises 75% ethanol; and
Extraction solution 6 (eluent) comprises 10 mM Tris-HCl (pH 8.0) and l mM EDTA (pH 8.0).

The preparation method comprises the following steps:
1. 1 ml of overnight culture of *E. coli* was placed in a centrifuge tube and mixed at 5000 rpm for 5 min, and the supernatant was sucked and discarded;
2. 200 µl of Extraction solution 1 was added to re-suspend the precipitate and 1 µl of RNA enzyme was added;
3. 400 µl of Extraction solution 2 was added, and the mixture was gently inverted up and down gently and mixed well;
4. 300 µl of Extraction solution 3 was added, and the mixture was inverted up and down, mixed well, and centrifuged at 12000 rpm for 10 minutes; then the supernatant was taken;
5. An equal volume of Extraction solution 4 was added, and the mixture was inverted up and down, mixed well, and left at -20°C for half an hour;
6. The resultant mixture was centrifuged at 12000 rpm for 10 minutes, and the supernatant was discarded;
7. 100 µl of Extraction solution 5 was added, and the mixture was rinsed once and dried at 37°C;
8. 30 µl of Extraction solution 6 was added and the mixture was mixed well; then, 10 µl of dissolved DNA was taken for electrophoresis;
   In the positive control, Extraction solution 3 was replaced with 3 M NaAc (pH 5.2) and the other reagents remained unchanged; In the negative control, Extraction solution 3 was replaced with water and the other reagents remained unchanged; and
9. An electrophoresis detection was performed, and the results were shown in Figure 3.

The results showed that when *E. coli* plasmids was extracted by using the above extraction solution, the extraction effect of tetrabutylammonium bromide was equivalent to that of NaAc indicating that tetrabutylammonium bromide could improve the precipitation recovery efficiency of *E. coli* plasmid.

### Example 4: Extraction and purification of E. coli genomic DNA

The present invention uses quaternary ammonium salts in the method for extraction of nucleic acid by phenol-chloroform, which could effectively extract and purify *E. coli* genomic DNA and comprise the following four components: Extraction solution 1, Extraction solution 2, Extraction solution 3, Extraction solution 4, and magnetic bead suspension.
Extraction solution 1 (lysis solution) comprises phosphate buffer (pH 7.0, 50 mM) and lysozyme (5 mg/ml);
Extraction solution 2 (lysis solution) comprises proteinase K (0.5 mg/ml);
Extraction solution 3 (chaotropic salt) comprises tetrabutylammonium bromide (2.5 M);
Extraction solution 4 (lysis solution) comprises phenol-chloroform-isopentanol (25:24:1);
Extraction solution 5 (binding solution) comprises absolute ethanol;
Extraction solution 6 (washing solution) comprises 75% ethanol; and
Extraction solution 7 (eluent) comprises 10 mM Tris-HCl (pH 8.0) and l mM EDTA (pH 8.0).

The preparation method includes the following steps:
1. 1 ml of overnight culture of *E. coli* competent cell was placed in a centrifuge tube and mixed at 5000 rpm for 5 min, and the supernatant was sucked and discarded;
2. 200 µl of Extraction solution 1 was added to re-suspend the precipitate, and the mixture was placed in a metal bath and incubated overnight at 37°C and 14000 rpm;
3. 100 µl of the lysis solution was taken and added with 10 µl of Extract solution 2, and the mixture was incubated at 56°C for 10 minutes;
4. 100 µl of Extraction solution 3 was added;
5. 200 µl of Extraction solution 4 was added, and the mixture was inverted up and down, mixed well, and centrifuged at 14000 rpm for 10 minutes; then, the supernatant was taken;
6. 200 µl of Extraction solution 5 was added, and the mixture was left at room temperature for half an hour and centrifuged at 14000 rpm for 10 minutes;
7. The supernatant was discarded, 100 µl of Extraction solution 6 was added, and the mixture was rinsed once and dried at 37°C;
8. 30 µl of Extraction solution 7 was added, and the mixture was shaken and mixed well.

### The above-mentioned dissolved DNA was amplified by PCR:

20 µl of amplification system comprises the following components: 10 µl 2 × PCR Mix, l µl of each primer (10 µM), 4 µl of the collected supernatant, and 4 µl pure water;
In the positive control, Extraction solution 3 was replaced with 5 M sodium chloride and the other reagents remained unchanged; in the negative control. Extraction solution 3 was replaced with water and the other reagents remained unchanged.
The thermal cycle is as follows: 95°C, 5 minutes; (95°C, 20 seconds; 60°C, 3 minutes) x 35 cycles

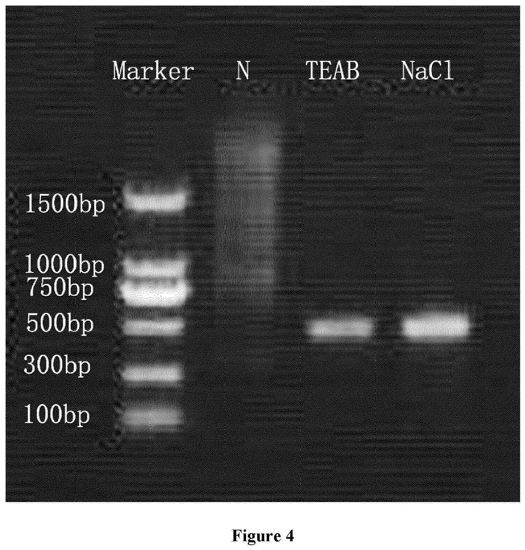

10 µl of the PCR products was taken and detected by electrophoresis, and the results were shown in Figure 4.

The results showed that when *E. coli* genome was extracted by using the above extraction solution, the extraction and amplification effects of tetrabutylammonium bromide were equivalent to those of NaCl, indicating that tetrabutylammonium bromide could improve the precipitation recovery efficiency of *E. coli* genome.

### Example 5: Extraction and purification of trace genomic DNA

The present invention could effectively extract and purify trace genomic DNA, and compared the extraction efficiencies of tetraethylammonium bromide and guanidine hydrochloride at the same concentration. The extraction reagents of the present invention comprise the following five components: Extraction solution 1, Extraction solution 2, Extraction solution 3, Extraction solution 4, and magnetic bead suspension.
Extraction solution 1 (lysis solution) comprises Tris-HCl (10 mM), EDTA (50 mM), SLS (1%), and proteinase K (0.5 mg/ml);
Extraction solution 2 (binding solution) comprises three components, wherein component A comprises 40% isopropanol, component B comprises 40% isopropanol and 1.6 M tetraethylammonium bromide, and component C comprises 1.6 M guanidine hydrochloride;
Extraction solution 3 (washing solution) comprises 80% ethanol.
Extraction solution 4 (washing solution) comprises 10 mM Tris-HCl (pH 8.0) and 1 mM EDTA (pH 8.0).

Magnetic bead suspension comprises superparamagnetic nanomagnetic beads dispersed in ultrapure water.

The preparation method includes the following steps:
l. 20 µl of 700-fold diluted human fresh blood was taken and placed into a centrifuge tube, and 400 µl of Extraction solution 1 was added to treat the blood at 56°C for 30 minutes. 2. 250 µl of Extraction solution 2 and 400 µg of magnetic bead suspension were added, and the mixture was shaken and mixed well at 800 rpm for 10 minutes.
3. The centrifuge tube was placed in a magnetic stand for magnetic separation and the liquid in the tube was sucked and discarded, 500 µl of Extraction solution 3 was added and the mixture was shaken and mixed well for 1 minute, then the supernatant was discarded after magnetic separation; step 3 was repeated once;
4. After the above magnetic beads were dried at room temperature until there was no obvious ethanol odor, 30 µl of Extraction solution 4 was added, and the mixture was left at 65°C for 10 minutes;
5. After magnetic separation, the supernatant was collected.

6 µl of the supernatant was taken and detected with NH18 STR amplification kit.

15 µl of amplification system comprises the following components: 6 µl 2.5 x PCR Mix, 3 µl NH18 primer mixture, and 6 µl of the collected supernatant, wherein in addition to the extracted trace samples, the template also comprise the standard 0.2 ng human genome as a positive control and Extraction solution 4 as a negative control.

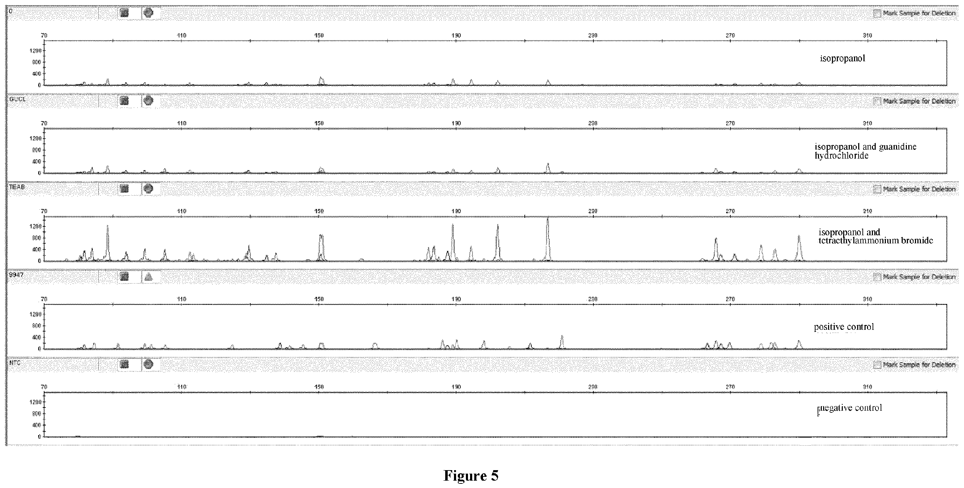

The thermal cycle is as follows: 95°C, 10 minutes; (95°C, 10 seconds; 59°C, 60 seconds) x 30 cycles; 60°C, 10 minutes. The results were shown in Figure 5.

The results showed that tetraethylammonium bromide could significantly improve the extraction efficiency for trace DNA, and the extraction efficiency of tetraethylammonium bromide is much higher than that of guanidine hydrochloride at the same concentration, indicating that tetrabutylammonium bromide could effectively extract and purify trace genomic DNA.

### Example 6: Comparison of CTAB and TEAB in the extraction of trace genomic DNA

Although cetyltrimethylammonium bromide (CTAB), which in fact belongs to quaternary ammonium salts, is often used in many methods for extracting plant genome, CTAB acts as a cationic surfactant in this method to facilitate lysis of plant cells, rather than as a chaotropic salt to facilitate binding of nucleic acid to adsorbing material or precipitation of nucleic acid. In addition, CTAB has a very low solubility in water (less than 0.1 M) and cannot be used alone to purify nucleic acids, and thus such a process also requires other chaotropic salts (such as sodium chloride, sodium acetate) in combination to separate and purify nucleic acids. Therefore, CTAB used in plant genome extraction is irrelevant to the technical solution of the present invention.

The present invention could effectively extract and purify trace genomic DNA and compared the extraction effects of CTAB and TEAB (tetrabutylammonium bromide). The extraction reagents of the present invention comprise the following five components: Extraction solution 1, Extraction solution 2, Extraction solution 3, Extraction solution 4, and magnetic bead suspension.
Extraction solution 1 (lysis solution) comprises Tris-HCl (50 mM final concentration), EDTA (36 mM final concentration), SLS (1% final concentration), and proteinase K (0.5 mg/ml);
Extraction solution 2 (binding solution) comprises two components (D, E), wherein component D comprises isopropanol (40% final concentration) and TEAB (1.6% M final concentration); component E comprises isopropanol (40% final concentration) and CTAB (2% stock solution concentration, 1.2% final concentration, which is already the maximum addition amount);
Extraction solution 3 (washing solution) comprises 80% ethanol;
Extraction solution 4 (eluent) comprises 10 mM Tris-HCl (pH 8.0) and 1 mM EDTA (pH 8.0);
Magnetic bead suspension comprises superparamagnetic nanomagnetic beads dispersed in ultrapure water.

The preparation method includes the following steps:
1. 30 µl of 700-fold diluted human fresh blood was taken and placed into a centrifuge tube, and 400 µl of Extraction solution 1 was added to the blood at 56°C for 30 minutes;
2. 250 µl of Extraction solution 2 and 400 µg magnetic bead suspension were added, and the mixture was shaken and mixed well at 800 rpm for 10 minutes;
3. The centrifuge tube was placed in a magnetic stand for magnetic separation and the liquid in the tube was sucked and discarded, 500 µl of Extraction solution 3 was added and the mixture was shaken and mixed well for 1 minute, then the supernatant was discarded after magnetic separation; step 3 was repeated once;
4. After the above magnetic beads were dried at room temperature until there was no obvious ethanol odor, 30 µl of Extraction solution 4 was added, and the mixture was treated at 65°C for 10 minutes;
5. After magnetic separation, the supernatant was collected.

6 µl of the supernatant was taken and detected with NH18 STR amplification kit.

15 µl of amplification system comprises the following components: 6 µl 2.5 x PCR Mix, 3 µl NH18 primer mixture, and 6 µl of the collected supernatant, wherein in addition to the extracted trace samples, the template also comprise the standard 0.2 ng human genome as a positive control and Extraction solution 4 as a negative control.

The thermal cycle is as follows: 95°C, 10 minutes; (95°C, 10 seconds; 59°C, 90 seconds) x 28 cycles; 60°C, 10 minutes.

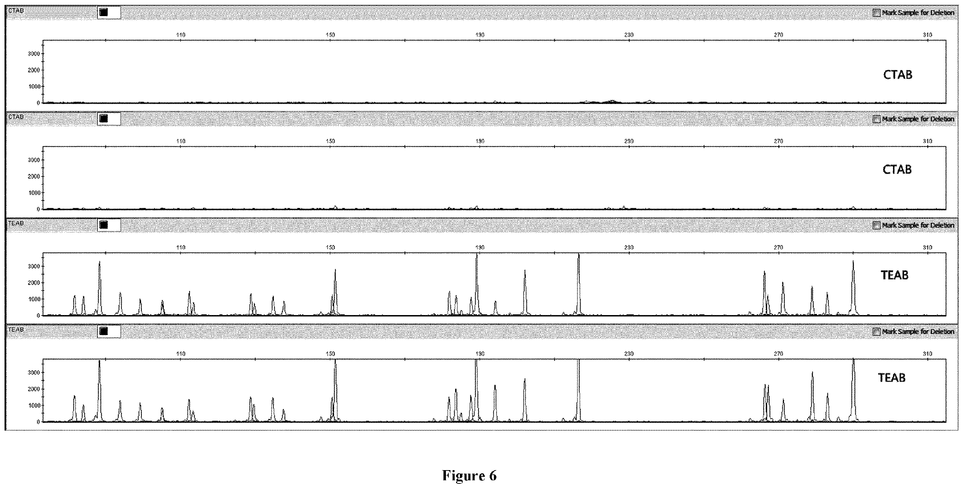

The results were shown in Figure 6. The results showed that TEAB could significantly improve the extraction efficiency for trace DNA, while CTAB could not substantially extract DNA.

### Example 7: Extraction of nucleic acid of MERS pseudovirus

The present invention aims to illustrate the influence of tetramethylammonium bromide on the extraction of nucleic acid of MERS pseudovirus, and the extraction reagents comprise the following three components: Extraction solution 1, Extraction solution 2 and Extraction solution 3.
Extraction solution 1 comprises MOPS (10 mM), isopropanol (50%), and tetramethylammonium bromide (1 M);
Extraction solution 2 comprises 80% ethanol;
Extraction solution 3 comprises ultrapure water.

The preparation method includes the following steps:
1. 10 µl of RNA of MERS pseudovirus was taken and added to a centrifuge tube containing 390 µl 0.9 % physiological saline, added with 400 µl of Extraction solution 1, and the mixture was transferred to a silicon membrane, left at room temperature for 5 min, and centrifuged at 8000 rpm for l min; then the waste liquid was discarded;
2. 500 µl of Extraction solution 2 was added, the mixture was left at room temperature for 3 min and centrifuged at 12000 rpm for l min, and the waste liquid was discarded; then, the empty tube was centrifuged at 12000 rpm for 2 min, and the waste liquid was discarded;
3. After the above silicon film was dried at room temperature until there was no obvious ethanol odor, 70 µl of Extraction solution 3 was added, the mixture was centrifuged at 12000 rpm for 1 min, and the centrifuged liquid was collected.

### The collected centrifuged liquid above was amplified by real-time PCR:

25 µl of amplification system comprises the following components: 12.5 µl 2 × PCR Mix, 1.5 µl each primer probe, 10 µl of the collected centrifugation liquid, and 1 µl pure water, wherein in addition to the extracted RNA of MERS pseudovirus, the templates also comprise 1.5 µl RNA of MERS pseudovirus at the same concentration as a positive control and Extraction solution 3 as a negative control.

The thermal cycle is as follows: 95°C, 10 minutes; (95°C, 15 seconds; 60°C, 45 seconds) x 40 cycles.

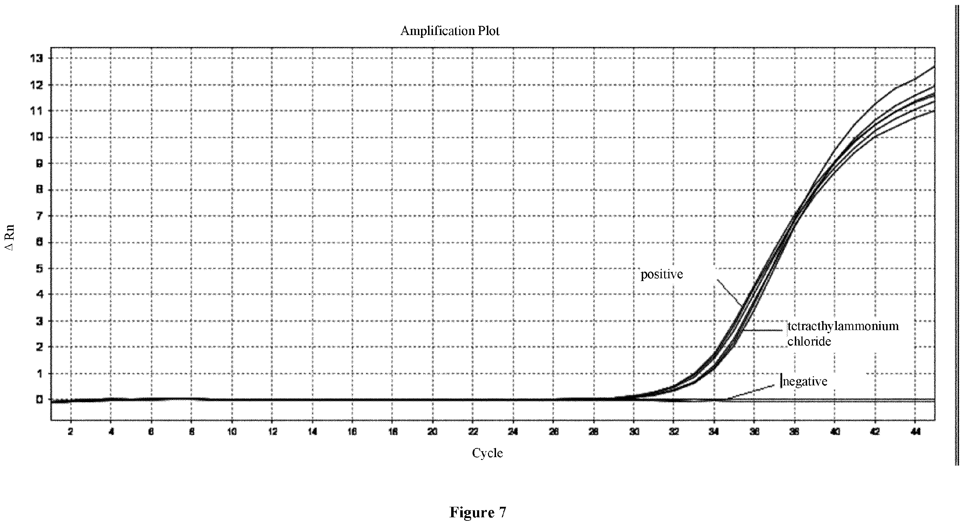

The results were shown in Figure 7. The results showed that addition of tetramethylammonium bromide in Extraction solution 1 was effective for the extraction of nucleic acid of MERS pseudovirus.

The above Examples are only the preferred embodiments of the present invention. It should be pointed out that, for those skilled in the art, various improvements and modifications can be made without departing from the principles of the present invention, and these improvements and modifications should be deemed as being within the protection scope of the present invention.

## Claims

1. Use of quaternary ammonium salt in nucleic acid extraction, wherein the quaternary ammonium salt has the structure as shown in formula I, 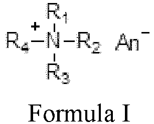 wherein An⁻ is an anion, and R₁, R₂, R₃, and R₄ are independently selected from alkyl groups.

2. The use according to claim 1, **characterized in that** the anion is a halogen anion.

3. The use according to claim 1 or 2, **characterized in that** the anion is a chloride ion or a bromide ion.

4. The use according to claim 1, **characterized in that** R₁, R₂, R₃, and R₄ are independently selected from C₁-C₅ alkyls.

5. The use according to claim 1, **characterized in that** R₁, R₂, R₃, and R₄ are independently selected from methyl, ethyl, propyl, butyl, and pentyl.

6. The use according to claim 1, **characterized in that** the quaternary ammonium salt acts as a chaotropic salt in nucleic acid extraction.

7. A nucleic acid extraction reagent, **characterized in that** a chaotropic salt in the nucleic acid extraction reagent is a quaternary ammonium salt having the structure as shown in Formula I of claim 1.

8. The nucleic acid extraction reagent according to claim 7, **characterized in that** the reagent comprises a quaternary ammonium salt having the structure as shown in Formula I of claim 1, and one or more components of a lysis solution, a binding solution, a washing solution, an eluent, and an adsorbing material; and the quaternary ammonium salt can be used independently as a component of the nucleic acid extraction reagent, or added to the lysis solution, or added to the binding solution.

9. The nucleic acid extraction reagent according to claim 8, **characterized in that** the lysis solution comprises one or more of protease, alkali and surfactant.

10. The nucleic acid extraction reagent according to claim 8, **characterized in that** the binding solution is isopropanol, absolute ethanol or polyethylene glycol.

11. A nucleic acid extraction method, **characterized in that** a quaternary ammonium salt having the structure as shown in Formula I of claim 1 is added as a chaotropic salt for extraction during the nucleic acid extraction process.

12. The extraction method according to claim 11, **characterized in** adding a lysis solution to the sample to be extracted for lysing the sample and releasing nucleic acid, and then adding a binding solution to bind the nucleic acid to the adsorbing material or precipitate the nucleic acid, and wherein the quaternary ammonium salt is added after the lysis or after the addition of the binding solution, or added to the lysis solution first and then added together, or added to the binding solution first and then added together.

13. The extraction method according to claim 12, **characterized in** further comprising washing the nucleic acid on the adsorbing material or the precipitated nucleic acid with a washing solution.

14. The extraction method according to claim 12 or 13, **characterized in** further comprising using an eluent to elute the nucleic acid on the adsorbing material, or using an eluent to dissolve the precipitated nucleic acid.
